# EUROPÄISCHE PATENTSCHRIFT

(11) **EP 3 939 711 B1**
(45) Veröffentlichungstag und Bekanntmachung des Hinweises auf die Patenterteilung: **12.07.2023**
(21) Anmeldenummer: 21173819.0
(22) Anmeldetag: 14.05.2021
(51) Int. Cl.: B08B 15/04, A61M 1/00

(54) **ABSAUGARM MIT FILTER**
SUCTION ARM WITH FILTER
BRAS D'ASPIRATION COMPRENANT UN FILTRE

(30) Priorität: 17.07.2020 DE 202020104146 U
(43) Veröffentlichungstag der Anmeldung: 19.01.2022
(73) Patentinhaber: TBH GmbH, 75334 Straubenhardt (DE)
(72) Erfinder: Farr, Fabian, 75196 Remchingen (DE)
(74) Vertreter: Durm Patentanwälte PartG mbB

(56) Entgegenhaltungen:
- EP-A1- 3 679 913
- US-A- 5 597 385
- US-B1- 6 406 454

## Beschreibung

Die vorliegende Erfindung betrifft einen Absaugarm für ein Absauggerät gemäß Anspruch 1, sowie eine Verwendung des Absaugarms mit einem medizinischen Absauggerät gemäß Anspruch 7.

Durch Prozesse am Arbeitsplatz können verschiedene Schadstoffe frei werden. Diese freiwerdenden Schadstoffe bergen ein Gesundheitsrisiko. Insbesondere können durch Mischung mit Gasen sowohl Flüssigkeiten als auch Feststoffe in Form von Aerosolen als luftgetragene Schwebeteilchen auftreten. Diese Aerosole sind unter anderem als Dampf, Nebel oder Rauch bekannt. Staubpartikel, insbesondere Feinstaub, können Schwebstoffe umfassen, deren Absetzzeiten mehrere Stunden betragen.

Es ist bekannt, derartige Schadstoffe mittels einer Absaugeinrichtung vom Arbeitsplatz zu entfernen. Hierbei kann ein Saugarm mit einem Absaugrohr verwendet werden. Das Absaugrohr kann mehrere Gelenke aufweisen, sodass eine am offenen Ende des Absaugrohrs befindliche Erfassungseinrichtung, beispielsweise ein Saugschirm, eine Saugdüse oder eine Ansaughaube, am Arbeitsplatz positioniert werden kann. Es gibt auch einfachere Konstruktionen ohne Gelenke.

Bei den bekannten Absaugvorrichtungen ist in der Regel ein Filtersystem vorgesehen, das sich im Absauggerät befindet. Angesaugte Luft mit darin befindlichen Schadstoffen wird dann mittels des Saugrohrs zum Absauggerät geführt und dort gefiltert. Nachteilig ist dabei, dass das Absaugrohr insbesondere in seinem inneren Bereich Schadstoffen ausgesetzt ist und dadurch verschmutzt.

Bei biowirksamen Schadstoffen wie beispielsweise Viren oder Bakterien kann es notwendig sein, das Absaugrohr von Zeit zu Zeit zu reinigen bzw. zu desinfizieren, um eine Gesundheitsgefährdung aufgrund der abgelagerten Schadstoffe auszuschließen.

Die Offenlegungsschrift EP 3 679 913 A1 betrifft einen Behandlungskopf für ein Unterdruckbehandlungsgerät und das Gerät. Der Behandlungskopf umfasst ein erstes Gehäuseteil. Das erste Gehäuseteil umfasst eine äußere Seitenfläche und eine Endfläche. Das erste Gehäuseteil definiert eine Aussparung für einen Filter und eine erste Öffnung der Aussparung zum Anordnen des Filters an der Aussparung. Die erste Öffnung wird teilweise durch die Endfläche und teilweise durch die äußere Seitenfläche definiert, um ein Entfernen des Filters aus der Aussparung zu erleichtern.

Die Offenlegungsschrift US 5 597 385 A betrifft ein Absaugsystem zum Entfernen von Laserrauchfahnen, die an einer Operationsstelle erzeugt werden, das einen handgehaltenen Absaugstab enthält. Der Absaugstab umfasst eine transparente zylindrische Ummantelung, die einen konischen Filter enthält, der in der proximalen Richtung divergiert. Ein Adapter, der an seinem proximalen Ende lösbar mit der Ummantelung gekoppelt ist, ermöglicht das Koppeln des Stabs mit Standard-Vakuumschläuchen, Verbindungsstücken und anderen Komponenten zum Abführen von Luft, die durch den Stab strömt. Der Stab wird so positioniert, dass sein distales Ende der Operationsstelle gegenüberliegt, wodurch an der Stelle erzeugte Schwaden und Gerüche sofort durch seine distale Öffnung in den Stab gezogen werden. Wenn Luft durch den Filter zu einer proximalen Öffnung des Stabs strömt, fängt der konische Filter die in der Luft befindlichen Verunreinigungen ein, um gegen eine Kontamination stromabwärts gelegener Komponenten zu schützen. Die abnehmbare Kupplung der Ummantelung ermöglicht einen bequemen Austausch des Filters. Bei Operationen, die ein höheres Kontaminationsrisiko darstellen, kann der gesamte Stab entsorgt werden, um eine sichere Entsorgung eingeschlossener Kontaminanten zu bewirken.

Die Patentschrift US 6 406 454 B1 betrifft ein Saugsondensystem zum Reinigen von Ablagerungen von einer Operationsstelle umfassend eine Sondenspitze, eine Filtereinheit in Fluidverbindung mit der Sondenspitze und eine Saugquelle in Fluidverbindung mit der Filtereinheit. Die Filtereinheit umfasst einen zylindrischen Filter mit einer inneren Kammer, die an der Saugquelle befestigt ist, und einer äußeren Oberfläche, die Schmutzpartikel ansammelt, die zu groß sind, um durch Löcher innerhalb des zylindrischen Filters zu gelangen. Die Filtereinheit umfasst auch einen Schieber mit einem Zylinder mit offenem Ende, der entlang der Außenseite des zylindrischen Filters geschoben wird, um angesammelte Schmutzpartikel zu entfernen und diese Schmutzpartikel in ein Reservoir innerhalb der Filtereinheit zu schieben. Der Schieber und die Sondenspitze können integrale Teile eines einzelnen Gleitelements sein.

Die vorliegende Erfindung stellt sich daher die Aufgabe, einen verbesserten Absaugarm für ein Absauggerät zu schaffen, insbesondere zur Verwendung mit einem medizinischen Absauggerät.

In einem ersten Aspekt betrifft die Erfindung einen Absaugarm für ein Absauggerät nach Anspruch 1, mit einem Saugrohr und einem Saugkopf, wobei der Absaugarm eine Saugstrecke definiert, die von einem Absaugbereich in einer Umgebung des Saugkopfes durch das Saugrohr zu dem Absauggerät verläuft, und wobei der Absaugarm ein austauschbares Filter umfasst, welches innerhalb der Saugstrecke angeordnet ist.

In einem zweiten Aspekt betrifft die Erfindung die Verwendung eines Absaugarms, wie im vorstehenden Absatz beschrieben, mit einem medizinischen Absauggerät.

Bevorzugte Ausgestaltungen der Erfindung werden in den abhängigen Ansprüchen beschrieben. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Mittels eines Saugrohrs kann eine hohe Absaugleistung erreicht werden, da das Saugrohr im Wesentlichen robust gegen Einwirkung eines Unterdrucks ist, der beim Saugen auftritt. Mittels eines Filters, das erfindungsgemäß innerhalb der Saugstrecke angeordnet ist, kann die Saugstrecke zumindest abschnittsweise schadstofffrei gehalten werden. Das austauschbare Filter ermöglicht es, mit nur kurzen Wartungspausen den Absaugarm für eine neue Anwendung vorzubereiten. Insbesondere können die Intervalle für die Reinigung und/oder Desinfektion des Saugrohrs verlängert werden. Es ist sogar möglich, dass durch den Einsatz des austauschbaren Filters in der Saugstrecke eine Desinfektion und/oder Reinigung des Saugrohrs komplett entfallen kann. Im medizinischen Bereich kann das einfache Wechseln des Filters das Vertrauen des Patienten und des behandelnden Arztes in das Absauggerät erhöhen; vor allem wenn der Filterwechsel vor den Augen des Patienten geschieht.

Das Filter ist in einem Filtergehäuse aufgenommen, wobei das Filtergehäuse dazu ausgebildet ist, das Filter und das Saugrohr luftdicht zu verbinden. Der Einsatz eines Filtergehäuses erleichtert einen Tausch des Filters. Ferner kann hierdurch technisch einfach eine korrekte Anordnung des Filters innerhalb der Saugstrecke erreicht werden. Die Ausbildung des Filtergehäuses derart, dass das Filter und das Saugrohr luftdicht verbunden sind, ermöglicht technisch einfach eine Erhöhung der Filterleistung des austauschbaren Filters. Das Filtergehäuse kann Gummidichtungen aufweisen. Das austauschbare Filter kann dadurch sehr einfach und kostengünstig hergestellt werden, da alle Mittel zur Herstellung einer Luftdichtheit am Filtergehäuse angeordnet sind.

Das Filtergehäuse weist einen Grundkörper auf, welcher mit dem Saugrohr verbunden ist, und einen abnehmbaren Deckel, um das Filtergehäuse zu verschließen. Der abnehmbare Deckel ermöglicht ein schnelles und einfaches Wechseln des austauschbaren Filters. Der mit dem Saugrohr verbundene Grundkörper ermöglicht technisch einfach eine luftdichte Verbindung zwischen Filtergehäuse und Saugrohr. Insbesondere kann der Grundkörper fest mit dem Saugrohr verbunden sein, beispielsweise angeklebt oder angeschweißt. Ferner kann bei der Fertigung des Grundkörpers das Saugrohr auch umspritzt werden, wenn der Grundköper im Spritzgussverfahren hergestellt wird.

Besonders bevorzugt weist der Grundkörper einen Verbindungsstutzen zum Anschluss an das Saugrohr auf. Vorzugsweise kann der Verbindungsstutzen einstückig mit dem Grundkörper ausgebildet sein. Das ermöglicht eine kostengünstige Herstellung von Grundkörper und Verbindungsstutzen als einziges Spritzgussteil. Eine solche einstückige Ausbildung von Saugrohr und Grundkörper des Filtergehäuses ist zum einen mechanisch sehr stabil und gewährleistet zum anderen absolute Luftdichtheit. Ferner ermöglicht ein Verbindungsstutzen, der vorzugsweise um den Umfang des Saugrohrs angeordnet ist, eine luftdichte Verbindung des Grundkörpers mit dem Saugrohr, ohne den Wirkungsquerschnitt des Saugrohrs zu verringern.

Vorteilhafterweise weist das Filtergehäuse ein Haltemittel auf, um den Deckel in einer geschlossenen Position zu halten. Durch einen Deckel mit einem Haltemittel kann das Filtergehäuse technisch einfach verschlossen und in der geschlossenen Position gehalten werden. Das Filter kann geschützt in dem Filtergehäuse aufgenommen werden. Insbesondere kann einem ungewollten Öffnen des Deckels und Verrutschen des Filters entgegengewirkt werden.

Besonders bevorzugt weist der Deckel einen Lufteinlass, vorzugsweise in Form eines Gitters, auf, um die angesaugte Luft in das Innere des Filtergehäuses zu leiten. Mittels eines Lufteinlasses im Deckel kann der Luftstrom der angesaugten Luft geleitet werden. Ein Lufteinlass in Form eines Gitters ermöglicht eine technisch einfache und günstige Herstellung des Deckels. Hierdurch kann ein bevorzugter Kompromiss aus Stabilität und Luftdurchlässigkeit des Deckels erreicht werden.

In einer weiteren vorteilhaften Ausgestaltung ist der Deckel des Filtergehäuses vom Absaugbereich aus zugänglich, um das Filter werkzeugfrei auszutauschen. Hierdurch kann das Filter effizient und schnell ausgetauscht werden. Insbesondere muss zum Austausch des Filters lediglich ein Bauteil, i. e. der Deckel des Filtergehäuses, angefasst und geöffnet werden. Eine Verwendung des Absaugarms ist komfortabel möglich.

Der Saugkopf umfasst eine Ansaughaube, in deren Mitte das Filtergehäuse sitzt. Eine solche Ansaughaube ermöglicht eine Modifikation des Absaugbereichs. Durch die Anordnung des Filtergehäuses in der Mitte der Ansaughaube kann eine effiziente Saugwirkung erreicht werden, wobei gleichzeitig eine gute Zugänglichkeit des Filtergehäuses, insbesondere zum Austausch des Filters, gegeben ist. Insbesondere kann durch diese vorteilhafte Anordnung praktisch der komplette Absaugarm schadstofffrei gehalten werden, da nur durch das Filter gefilterte Luft in das Saugrohr gelangt.

Die Absaughabe ist mit dem Grundkörper verbunden. Es versteht sich, dass eine einstückige Verbindung vorgesehen sein kann, also dass der Grundkörper einstückig mit der Absaughaube ausgebildet ist. Beispielsweise kann der Grundkörper zusammen mit der Absaughaube ein einziges Spritzgussteil sein. Es versteht sich ferner, dass die Absaughaube auch mittels Kleben, Schrauben oder anderen bekannten Verbindungsarten mit dem Grundkörper verbunden werden kann. Insbesondere kann hier eine lösbare Verbindung vorgesehen sein, um verschiedene Absaughauben mit dem gleichen Grundkörper zu kombinieren.

Der Deckel des Filtergehäuses weist ein Rastmittel, insbesondere zwei Rastmittel, auf, mittels derer er am Grundkörper lösbar befestigt ist. Mittels eines solchen Rastmittels kann ein sicherer Sitz des Deckels am Grundkörper gewährleistet sein. Insbesondere ist es denkbar, dass das Rastmittel den Deckel kraftbeaufschlagt auf den Grundkörper drückt, sodass eine sichere und feste Verbindung zwischen Grundkörper und Deckel erreicht wird.

Das Rastmittel weist ein Federelement auf, welches sich durch eine Ausnehmung in der Ansaughaube hindurch erstreckt, um mit dem Grundkörper zu verrasten. Durch ein solches Federelement bzw. zwei Federelemente ist eine einfache, verliersichere Anordnung des Deckels an dem Grundkörper möglich. Die Ausnehmungen an der Ansaughaube ermöglichen es, das Federelement außerhalb des Absaugbereichs von Hand zu lösen. Die Wahrscheinlichkeit einer Kontamination beim Lösen des Federelements ist dadurch verringert. Durch die Ausnehmungen an der Ansaughaube ist es möglich, dieselbe Ansaughabe für Absaugarme mit Filtergehäuse und ohne Filtergehäuse zu verwenden. Die Ansaughabe kann folglich in großer Stückzahl kostengünstig hergestellt werden.

Unter Absaugbereich ist der Bereich zu verstehen, auf den eine Saugwirkung mittels des Absauggeräts ausgeübt wird. Es versteht sich, dass der Absaugbereich abhängig vom jeweiligen Schadstoff und abhängig von der jeweiligen Erfassungseinrichtung, wie beispielsweise Saugdüse, Saugschirm, Saugglocke, ist. Insbesondere kann der Absaugbereich als ein Bereich verstanden werden, in dem ein vordefinierter Prozentsatz eines bestimmten Schadstoffes aus der Luft entfernt wird.

Ein Saugrohr ist vorliegend als Rohr zu verstehen, das eine Verbindung zwischen Saugkopf und Absauggerät schafft, wobei das Saugrohr eine Saugwirkung des Absauggeräts zu dem Absaugbereich leitet.

Ein Ausführungsbeispiel der Erfindung wird nachfolgend im Zusammenhang mit den beiliegenden Zeichnungen näher beschrieben und erläutert. Es zeigen:
- Figur 1: eine schematische Darstellung eines Absaugarms;
- Figur 2: eine vereinfachte perspektivische Darstellung eines Absauggeräts mit Absaugarm;
- Figur 3: allein den Saugkopf des Absaugarms von Fig. 2;
- Figur 4: allein die Ansaughaube des Saugkopfes von Fig. 3;
- Figur 5: allein das Filtergehäuse des Saugkopfes von Fig. 3, in geschlossenem Zustand; und
- Figur 6: das geöffnete Filtergehäuse mit Filter, in einer Explosionsdarstellung.

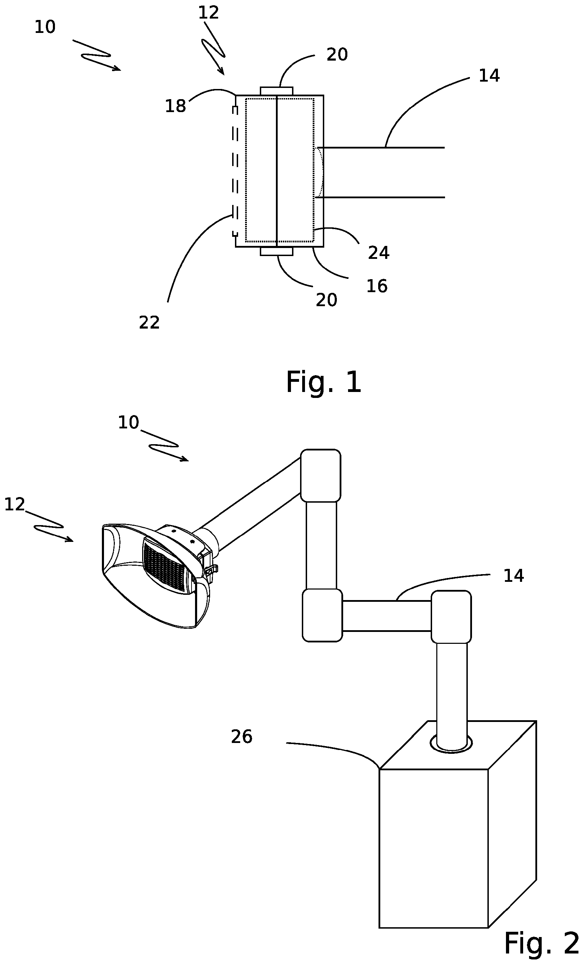

Figur 1 zeigt schematisch einen Absaugarm 10 mit einem Saugkopf 12, der an einem Saugrohr 14 angeordnet ist. Der Saugkopf 12 ist am offenen Ende des Saugrohrs 14 angeordnet.

Der Saugkopf 12 weist ein Filtergehäuse mit einem Grundkörper 16 und einem Deckel 18 auf. Der Deckel 18 ist mit Haltemitteln 20 an dem Grundkörper 16 angeordnet. Zudem weist der Deckel 18 einen Lufteinlass 22 auf, um angesaugte Luft ins Innere des Filtergehäuses zu leiten.

Im Inneren des Filtergehäuses sitzt ein Filter 24. Das Filtergehäuse und insbesondere der Grundkörper 16 ist dazu ausgebildet, das Filter 24 luftdicht mit dem Saugrohr 14 zu verbinden, sodass durch das Filter 24 gefilterte Luft ins Innere des Saugrohrs 14 gelangt. Durch den Lufteinlass 22 strömt die angesaugte Luft ins Innere des Filtergehäuses. Der Lufteinlass 22 kann dabei den Luftstrom vorteilhaft zum Filter 24 leiten.

In Figur 2 ist ein Absauggerät 26 mit einem Absaugarm 10 gezeigt.

Der Absaugarm 10 weist einen Saugkopf 12 auf, der mittels des Saugrohrs 14 mit dem Absauggerät 26 verbunden ist. Der Saugkopf 12 hat eine Erfassungseinrichtung in Form einer Ansaughaube. Das Filtergehäuse ist mittig in der Erfassungseinrichtung angeordnet. Das Absauggerät 26 mit dem Absaugarm 10 kann beispielsweise bei der Behandlung eines Patienten verwendet werden, um Schmutz, Schadstoffe, Viren und Bakterien zuverlässig abzusaugen. Mittels des Saugkopfes 12 kann gewährleistet werden, dass das Saugrohr 14 im Wesentlichen sauber und keimfrei bleibt. Das Filter, das mittig im Saugkopf 12 angeordnet ist, kann nach der Behandlung einfach gewechselt werden.

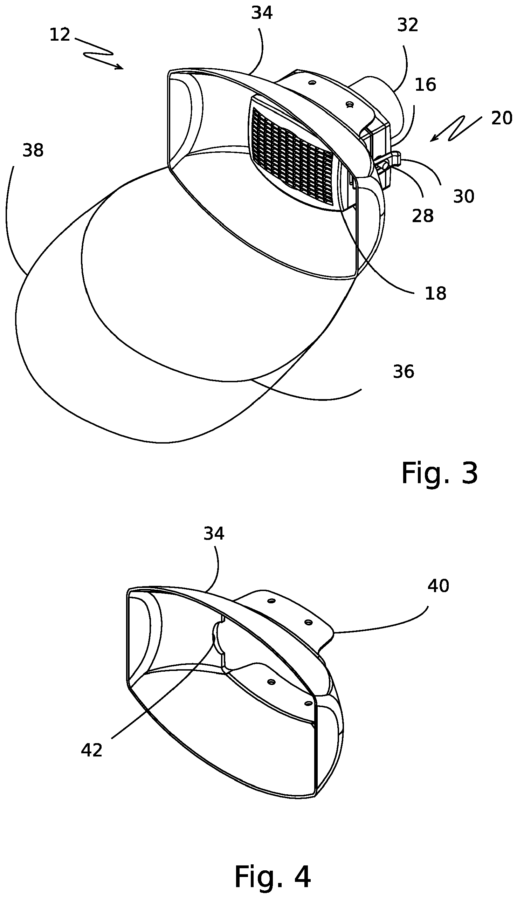

In Figur 3 ist der Saugkopf 12 detaillierter dargestellt. In dem gezeigten Beispiel umfasst das Haltemittel 20 einen Stift 28, der am Grundkörper 16 angeordnet ist, und ein Rastmittel in Form eines Federelements 30, das von dem Grundkörper 16 weg federnd ausgebildet ist und eine Art Lasche bildet, die sich um den Stift 28 legen kann. Der Stift 28 wird folglich in der Lasche aufgenommen, wobei das Federelement 30 aufgrund der Federwirkung einrastet und so den Deckel 18 am Grundkörper 16 fixiert. Vorteilhafterweise ist das Federelement 30 an dem vom Deckel 18 wegweisenden Ende leicht schräg ausgebildet, sodass beim Aufschieben des Deckels 18 auf den Grundkörper 16 das Federelement 30 aufgrund der Abschrägung vom Stift 28 entgegen der Federwirkung nach außen gedrückt wird und bei weiterem Einschieben des Deckels 18 in Richtung des Grundkörpers 16 wieder zurückfedert, sobald der Stift 28 eine Aussparung im Federelement 30 erreicht. Das Federelement 30 hält den Deckel 18 in einer geschlossenen Position.

Zur Befestigung am Saugrohr 14 (vgl. Fig. 1) weist der Grundkörper 16 einen Verbindungsstutzen 32 auf. Hier ist der Verbindungsstutzen 32 als runder Flansch ausgebildet, sodass der Grundkörper 16 auf das Saugrohr 14 aufgesteckt werden kann. Es versteht sich, dass der Verbindungsstutzen 32 eine Dichtung und/oder eine Klebeschicht aufweisen kann, um eine feste und dichte Verbindung mit dem Saugrohr 14 zu erreichen.

Der Saugkopf 12 umfasst ferner eine Ansaughaube 34. Die Ansaughaube 34 kann an dem Filtergehäuse befestigt werden, beispielsweise durch Schrauben, Kleben, Einrasten und/oder Aufschrauben. Mittels der Ansaughaube 34 wird der Absaugbereich des Saugkopfes 12 modifiziert. Beispielhaft ist in der Figur 3 ein erster Absaugbereich 36 und ein zweiter Absaugbereich 38 gezeigt. Die Absaugbereiche 36, 38 können beispielsweise derart definiert sein, dass im ersten Absaugbereich 36 99,9% eines Schadstoffes abgesaugt werden, wohingegen im zweiten Absaugbereich 38 nur 95% des Schadstoffes abgesaugt werden.

Es versteht sich, dass der Absaugbereich unter Anderem abhängig ist von einer Ausrichtung des Saugkopfes 12, der Ausbildung der Erfassungseinrichtung, wie beispielsweise der Ansaughaube 34, und des abzusaugenden Schadstoffs. Insbesondere kann ein Absaugbereich sowohl eine Absaugfläche als auch ein Absaugvolumen umfassen. Die Erfassungseinrichtung kann alternativ auch als Saugdüse ausgebildet sein.

In Figur 4 ist die Ansaughaube 34 detailliert dargestellt. Die Ansaughaube 34 weist einen Verbindungsabschnitt auf, der in Form von zwei Verbindungsstücken 40 ausgebildet ist, welche sich in Richtung des Saugrohrs erstrecken. Vorzugsweise weist das Verbindungsstück 40 an der dem Filtergehäuse zugewandten Seite eine zu der Außenseite des Filtergehäuses korrespondierende Form auf. Der Verbindungsabschnitt 40 hat zwei Bohrungen, um die Ansaughaube 34 an dem Filtergehäuse anzuschrauben. Es versteht sich, dass das Verbindungsstück 40 auch elastisch ausgebildet sein kann, wobei das Verbindungsstück 40 nach außen zurückweichen kann und mit entsprechenden Stiften am Filtergehäuse rastend befestigt werden kann.

Die Ansaughaube 34 weist ferner Ausnehmungen 42 auf. Die Ausnehmungen 42 sind derart ausgebildet, dass das Federelement 30 des Deckels 18 durch die Ausnehmungen 42 hindurchgeführt werden und auf der Rückseite der Ansaughaube 34, wie in Figur 3 gezeigt, vom Stift 28 gehalten werden kann.

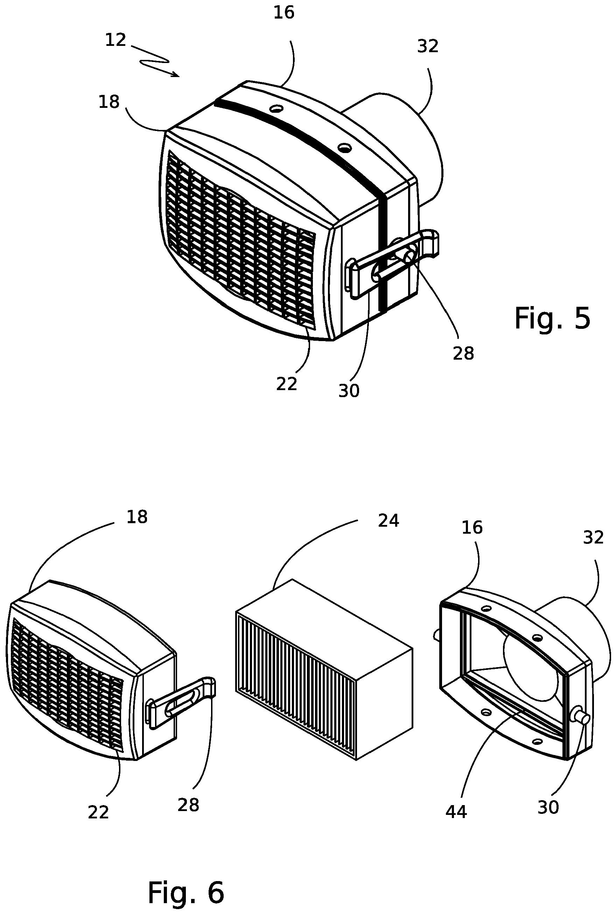

In Figur 5 ist das Filtergehäuse ohne Ansaughaube 34 zu sehen. Gleiche Bezugszeichen beziehen sich auf gleiche Merkmale und werden nicht erneut erläutert. Anhand Figur 5 kann die Wirkungsweise des Rastmittels in Form eines Federelements 30 genauer erläutert werden. Das Federelement 30 umfasst einen Arm bzw. eine Lasche, die eine Aussparung aufweist, wobei der Stift 28 in die Aussparung des Federelements 30 greifen kann. Hierdurch kann der Deckel 18 lösbar, aber fest am Grundkörper 16 befestigt werden. Das Federelement 30 kann nach außen federn. Beispielsweise kann das durch eine Materialschwächung der Verbindung zwischen Federelement 30 und Deckel 18 erreicht werden. Es versteht sich, dass auch eine Metallverbindung oder eine Feder vorgesehen sein kann, um ein federndes Zurückweichen des Federelements 30 zu ermöglichen. Wie bereits oben beschrieben, weist das Federelement 30 an dem zum Saugrohr weisenden Ende eine Krümmung auf, sodass beim Aufschieben des Deckels 18 das Federelement 30 aufgrund der Wechselwirkung der Krümmung des Federelements 30 mit dem Stift 28 nach außen gedrückt wird. Beim weiteren Einschieben des Deckels 18 in Richtung des Saugrohrs schnappt das Federelement 30 aufgrund der Federwirkung nach innen, wenn der Stift 28 in die Aussparung im Rastmittel 30 eindringt. Hierdurch kann der Deckel 18 durch einfaches Aufschieben von vorne, also vom Absaugbereich her, an dem Grundkörper 16 angeordnet werden. Durch Biegen des Federelements 30 entgegen der Federwirkung, also Ziehen nach außen, kann die Rastverbindung gelöst werden und der Deckel 18 vom Grundkörper 16 abgenommen werden. Bei aufgesetzter Ansaughaube 34 kann das Betätigen des Federelements 30 hinter der Ansaughaube 34 erfolgen.

Der Grundkörper 16 weist ferner zwei Bohrungen auf, die dazu vorgesehen sind, den Grundkörper 16 mit der Ansaughaube 34 zu verbinden.

Figur 6 ist eine Explosionszeichnung des Filtergehäuses gemäß Figur 5. Das Filter 24 ist quaderförmig ausgebildet. Das Filter 24 kann in den Grundkörper 16 eingesetzt werden. Der Grundkörper 16 weist eine Anlegekante 44 auf, um einen sicheren Sitz des Filters 24 in einer vordefinierten Position im Filtergehäuse zu ermöglichen. Die Anlegekante 44 kann eine Dichtung, insbesondere eine Gummidichtung, aufweisen, um eine luftdichte Anbindung des Filters 24 an den Grundkörper 16 zu ermöglichen. Durch diese Ausgestaltung ist es möglich, das Filter 24 werkzeugfrei und schnell aus dem Filtergehäuse zu entnehmen und gegen ein frisches Filter auszutauschen.

In den Schutzansprüchen schließen die Wörter "umfassen" und "mit" nicht das Vorhandensein weiterer Elemente aus. Der undefinierte Artikel "ein" oder "eine" schließt nicht das Vorhandensein einer Mehrzahl aus. Ein einzelnes Element oder eine einzelne Einheit kann die Funktionen mehrerer der in den Schutzansprüchen genannten Einheiten ausführen.

### Bezugszeichen

- 10: Absaugarm
- 12: Saugkopf
- 14: Saugrohr
- 16: Grundkörper
- 18: Deckel

- 20: Haltemittel
- 22: Lufteinlass
- 24: Filter
- 26: Absauggerät
- 28: Stift

- 30: Federelement
- 32: Verbindungsstutzen
- 34: Ansaughaube
- 36: erster Absaugbereich
- 38: zweiter Absaugbereich

- 40: Verbindungsstück
- 42: Ausnehmung
- 44: Anlegekante

## Patentansprüche

1. Absaugarm (10) für ein Absauggerät (26) mit einem Saugrohr (14) und einem Saugkopf (12), wobei
der Absaugarm (10) eine Saugstrecke definiert, die von einem Absaugbereich (36, 38) in einer Umgebung des Saugkopfes (12) durch das Saugrohr (14) zu dem Absauggerät (26) verläuft,
der Absaugarm (10) ein austauschbares Filter (24) umfasst, welches innerhalb der Saugstrecke angeordnet ist;
das Filter (24) in einem Filtergehäuse aufgenommen ist, das dazu ausgebildet ist, das Filter (24) und das Saugrohr (14) luftdicht zu verbinden;
das Filtergehäuse einen Grundkörper (16) aufweist, welcher mit dem Saugrohr (14) verbunden ist, und einen abnehmbaren Deckel (18), um das Filtergehäuse zu verschließen;
der Saugkopf (12) eine Ansaughaube (34) umfasst, die mit dem Grundkörper (16) verbunden ist und in deren Mitte das Filtergehäuse sitzt;
der Deckel (18) des Filtergehäuses mit einem Rastmittel an dem Grundkörper (16) lösbar befestigt ist;
das Rastmittel ein Federelement am Deckel umfasst, welches sich durch eine Ausnehmung (42) in der Ansaughaube (34) hindurch erstreckt, um mit dem Grundkörper (16) zu verrasten, sodass das Federelement außerhalb des Absaugbereichs von Hand zu lösen ist.

2. Absaugarm (10) nach dem vorstehenden Anspruch, wobei der Grundkörper (16) einen Verbindungsstutzen (32) zum Anschluss an das Saugrohr (14) aufweist.

3. Absaugarm (10) nach einem der vorstehenden Ansprüche, wobei das Filtergehäuse ein Haltemittel (20) aufweist, um den Deckel (18) in einer geschlossenen Position zu halten.

4. Absaugarm (10) nach einem der vorstehenden Ansprüche, wobei der Deckel (18) einen Lufteinlass (22), vorzugsweise in Form eines Gitters, aufweist, um die angesaugte Luft in das Innere des Filtergehäuses zu leiten.

5. Absaugarm (10) nach einem der vorstehenden Ansprüche, wobei der Deckel (18) des Filtergehäuses vom Absaugbereich (36, 38) aus zugänglich ist, um das Filter (24) werkzeugfrei auszutauschen.

6. Absaugarm (10) nach einem der vorstehenden Ansprüche, wobei der Deckel (18) des Filtergehäuses mit zwei Rastmitteln an dem Grundkörper (16) lösbar befestigt ist.

7. Verwendung des Absaugarms (10) nach einem der vorstehenden Ansprüche mit einem medizinischen Absauggerät (26).

## Claims

1. An extraction arm (10) for an extraction device (26) having a suction pipe (14) and a suction head (12), wherein
the extraction arm (10) defines a suction path that extends from an extraction region (36, 38) in the surroundings of the suction head (12) through the suction pipe (14) to the extraction device (26);
the extraction arm (10) comprises an exchangeable filter (24) which is arranged within the suction path;
the filter (24) is accommodated in a filter housing and the filter housing is designed to connect the filter (24) and the suction pipe (14) in an airtight manner;
the filter housing has a main body (16) which is connected to the suction pipe (14) and a removable cover (18) for closing the filter housing;
the suction head (12) comprises an extraction hood (34), which is connected to the main body (16) and in the center of which the filter housing is located;
the cover (18) of the filter housing is releasably attached to the main body (16) by a latching means;
the latching means comprises a spring element on the cover, which spring element extends through a recess (42) in the extraction hood (34) in order to latch with the main body (16), so that the spring element can be released by hand outside the extraction region.

2. The extraction arm (10) according to the preceding claim, wherein the main body (16) has a connecting piece (32) for connection to the suction pipe (14).

3. The extraction arm (10) according to any one of the preceding claims, wherein the filter housing has a holding means (20) for holding the cover (18) in a closed position.

4. The extraction arm (10) according to any one of the preceding claims, wherein the cover (18) has an air inlet (22), preferably in the form of a grille, in order to guide the extracted air into the interior of the filter housing.

5. The extraction arm (10) according to any one of the preceding claims, wherein the cover (18) of the filter housing is accessible from the extraction region (36, 38) in order to replace the filter (24) without using tools.

6. The extraction arm (10) according to any one of the preceding claims, wherein the cover (18) of the filter housing is releasably attached to the main body (16) by two latching means.

7. Applying the extraction arm (10) according to any one of the preceding claims with a medical extraction device (26).

## Revendications

1. Bras d'aspiration (10) pour un dispositif d'aspiration (26) avec un tuyau d'aspiration (14) et une tête d'aspiration (12), dans lequel
le bras d'aspiration (10) définit un trajet d'aspiration qui s'étend depuis une zone d'aspiration (36, 38) dans un environnement de la tête d'aspiration (12) à travers le tuyau d'aspiration (14) jusqu'au dispositif d'aspiration (26);
le bras d'aspiration (10) comprenant un filtre remplaçable (24) qui est disposé dans le trajet d'aspiration;
le filtre (24) est logé dans un boîtier de filtre et le boîtier de filtre est conçu pour relier le filtre (24) et le tuyau d'aspiration (14) de manière étanche à l'air;
le boîtier de filtre comprend un corps de base (16) qui est relié au tuyau d'aspiration (14) et un couvercle désaccouplable (18) pour fermer le boîtier de filtre;
la tête d'aspiration (12) comprend un capot d'aspiration (34) qui est relié au corps de base (16) et au centre du quel le boîtier de filtre est logé;
le couvercle (18) du boîtier de filtre étant fixé de manière désaccouplable au corps de base (16) avec un moyen d'encliquetage;
le moyen d'encliquetage comprend un élément à ressort sur le couvercle qui s'étend à travers un passage (42) dans le capot d'aspiration (34) afin de s'enclencher avec le corps de base (16), de sorte que l'élément à ressort peut être libéré à la main en dehors de la zone d'aspiration.

2. Bras d'aspiration (10) selon la revendication précédente, dans lequel le corps de base (16) comporte un manchon de connexion (32) pour le raccordement au tuyau d'aspiration (14).

3. Bras d'aspiration (10) selon l'une quelconque des revendications précédentes, dans lequel le boîtier de filtre comporte un moyen de fixation (20) pour fixer le couvercle (18) dans une position fermée;

4. Bras d'aspiration (10) selon l'une quelconque des revendications précédentes, dans lequel le couvercle (18) présente une entrée d'air (22), de préférence sous forme de grille, pour diriger l'air aspiré à l'intérieur du boîtier de filtre.

5. Bras d'aspiration (10) selon l'une quelconque des revendications précédentes, le couvercle (18) du boîtier de filtre étant accessible depuis la zone d'aspiration (36, 38) pour échanger le filtre (24) sans outils.

6. Bras d'aspiration (10) selon l'une quelconque des revendications précédentes, le couvercle (18) du boîtier de filtre étant fixé de manière désaccouplable au corps de base (16) avec deux moyens d'encliquetage.

7. Utilisation du bras d'aspiration (10) selon l'une quelconque des revendications précédentes avec un dispositif médical d'aspiration (26).
